(19) Europäisches Patentamt
European Patent Office
Office européen des brevets

(11) Publication number: 0 213 313 A2

(12) **EUROPEAN PATENT APPLICATION**

(21) Application number: 86108910.0

(22) Date of filing: 01.07.86

(51) Int. Cl.[4]: C 07 D 307/54
C 07 D 307/42, C 07 D 307/32
C 07 C 59/90, C 07 C 69/738
C 07 C 177/00

(30) Priority: 29.07.85 US 760023

(43) Date of publication of application:
11.03.87 Bulletin 87/11

(84) Designated Contracting States:
BE CH DE FR GB IT LI NL SE

(71) Applicant: AMERICAN CYANAMID COMPANY
1937 West Main Street P.O. Box 60
Stamford Connecticut 06904-0060(US)

(72) Inventor: Floyd, Middleton Brawner, Jr.
5 Babbling Brook Lane
Suffern New York 10901(US)

(74) Representative: Wächtershäuser, Günter, Dr.
Tal 29
D-8000 München 2(DE)

(54) **Precursors and synthesis of methyl-9-oxo-11 alpha, 16-dihydroxy-16-vinyl-5-cis-13-trans prostadienoates.**

(57) Novel precursors for the synthesis of methyl-9-oxo-11α, 16-dihydroxy-16-vinyl-5-*cis*-13-*trans* prostadienoates having the formula

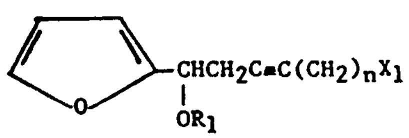

(2)

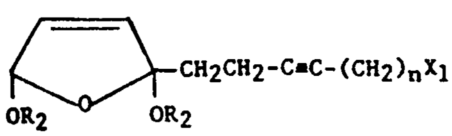

(3)

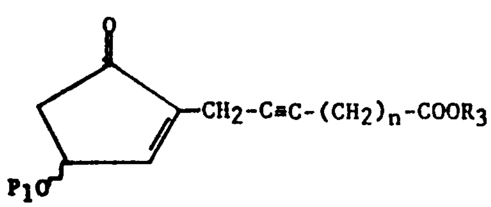

(4a)

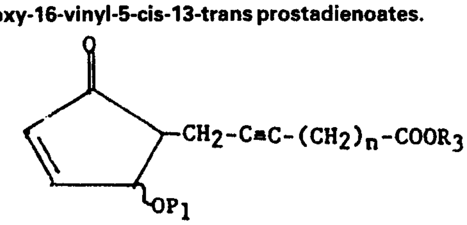

(4b)

wherein $R_1$ is H or $C_1$-$C_4$ alkyl; $R_2$ is $C_1$-$C_4$ alkyl, $R_3$ is H, $C_1$-$C_4$ trialkylsilyl or $C_1$-$C_6$ alkyl; $X_1$ is halogen, cyano, $C_1$-$C_4$ alkoxycarbonyl, carboxy or tri ($C_1$-$C_4$ alkoxy)methyl; n is 2-4 inclusive; and $P_1$ is H or a blocking or protective group; congeners and racemic mixture of these compounds and processes of synthesizing them.

EP 0 213 313 A2

Croydon Printing Company Ltd.

Title: PRECURSORS AND SYNTHESIS OF METHYL-9-OXO-11α,16-DIHYDROXY-16-VINYL-5-CIS-13-TRANS-PROSTADIENOATES

## FIELD OF THE INVENTION

This invention relates to novel compounds which are advantageously used as precursors in the synthesis of methyl-9-oxo-11α,16-dihydroxy-16-vinyl-5-*cis*-13-*trans*-prostadienoates, their congeners and racemic mixtures thereof. These prostadienoates are pharmacologically active, for instance, as hypotensive agents and as vasodilators. One of such prostadienoates having vasodilator and hypotensive activity, namely methyl 9-oxo-11α, 16-dihydroxy-16-vinylprosta-5-*cis*-13-*trans*-dienoate, having the structure

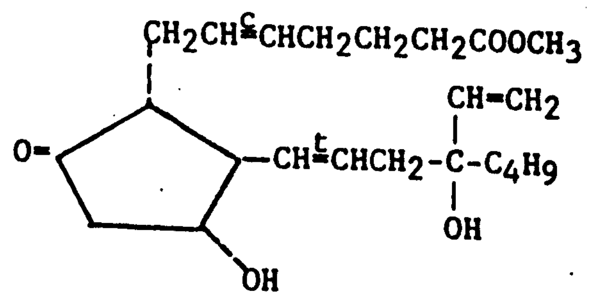

(1)

has been described in U. S. Patents 4,198,521 and 4,311,707 and by J. E. Birnbaum, *et al*., J. Med. Chem., 25, 492 (1982).

0213313

This invention also relates to novel processes of synthesizing new compounds which are precursors to the pharmacologically active compounds cited above.

The novel compounds and processes of the invention provide a simpler, more rapid and economical means of producing pharmacologically active prostadienoates of greatly increased purity as compared to the compounds and processes used heretofore.

## BRIEF SUMMARY OF THE INVENTION

The novel precursor compounds of this invention include the acetylenic furan derivatives represented by the formula

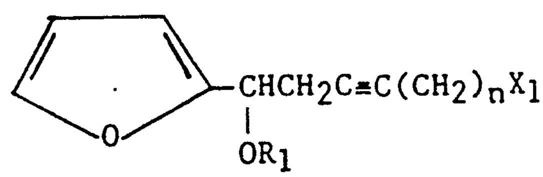

(2)

wherein $R_1$ is selected from the group consisting of hydrogen and $C_1$-$C_4$ alkyl; $X_1$ is selected from the group consisting of halogen, cyano, $C_1$-$C_4$ alkoxycarbonyl, carboxy and tri-($C_1$-$C_4$ alkoxy)methyl; and n is an integer 2-4 inclusive.

The novel precursor compounds also include the class of furan derivatives represented by the formula

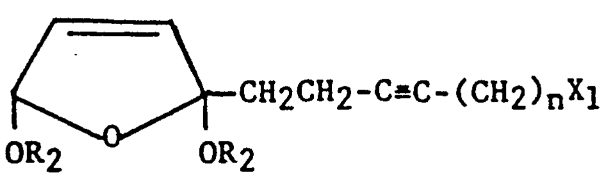

(3)

wherein $R_2$ is selected from the group consisting of $C_1$-$C_4$ alkyl and n and $X_1$ are as hereinabove defined.

From the compounds described hereinabove, one may obtain, by processes described below, additional novel compounds which are cyclopentenones of the class represented by the formulae

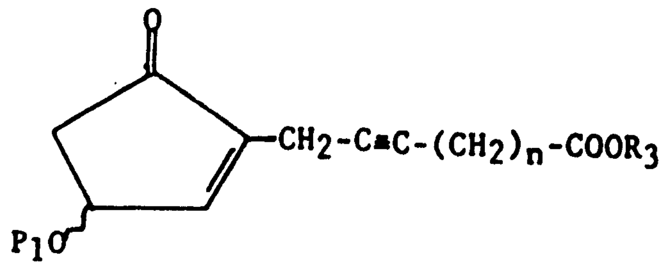

(4a)

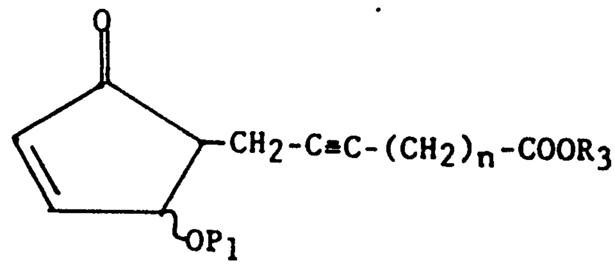

(4b)

wherein $R_3$ is a monovalent radical selected from the group consisting of hydrogen, $C_1$-$C_4$ trialkylsilyl and $C_1$-$C_6$ alkyl; n is an integer 2-4 inclusive; $P_1$ is a monovalent radical selected from the group consisting of hydrogen and $P_2$; and $P_2$ is a blocking or protective group which is stable under the conditions of the catalytic hydrogenation of the triple bond, as described hereinafter, or under the conditions of the 1,4 conjugate addition process to which compound (4a) is to be subjected, as described hereinafter, and which can thereafter be removed, usually by a mild acid treatment, without disruption of the prostaglandin product of which it forms a part. Such protective groups are well-known in the art and may be, for instance, tetrahydropyranyl, trialkylsilyl, such as trimethylsilyl or dimethyl-t-butylsilyl, or alpha-alkoxy ethyl.

It is significant that the novel intermediate of formula (4a), where P is H, n is 3 and $R_3$ is hydrogen, is crystalline. Its crystalline structure enhances and simplifies purification, thereby providing a more economical process of preparing a product of high purity.

Broadly described, the novel processes of this invention include the preparation of compounds of formula (2) by reacting an alkali metal acetylide of formula (5), wherein $R_4$ is $C_1$-$C_4$ alkyl or the alkali metal cation represented by M when M is a lithium, sodium or potassium cation; with a substituted alkyl bromide or iodide of formula (6)

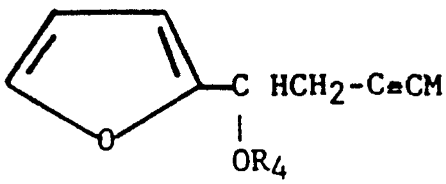

+ $Y-(CH_2)_n-X_2$

(6)

(5)

wherein Y is bromo or iodo; n is an integer 2-4 inclusive and $X_2$ is selected from the group consisting of halogen and tri-($C_1$-$C_4$ alkoxy)methyl.

Alternatively the compound (7)

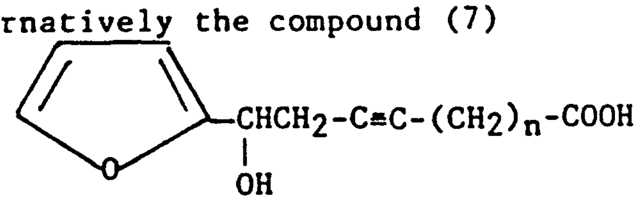

(7)

where n is an integer 2-4 inclusive, may be prepared by reacting 2-(2-furyl)oxirane [Zh. Organ.Khim., 1, 539 (1965)] with a di-(alkali metal)acetylide carboxylate (8)

$$MC{\equiv}C-(CH_2)_n-COOM$$

(8)

where M and n are as defined hereinabove.

The novel processes also include the preparation of compounds of formula (3) by reacting an alkali metal acetylide (9)

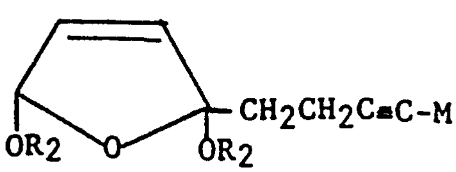

(9)

wherein $R_2$ and M are as defined hereinabove, with a substituted alkyl bromide or iodide defined by formula (6).

The compound of formula (2) or (3) is then subjected to an acid-catalyzed hydrolysis and isomerization, and, optionally, esterification, to form, via the intermediate 3-hydroxy isomer (4b), the 4-hydroxycyclopentenone compound (4a), wherein $P_1$ is hydrogen.

The 4-hydroxy group of the cyclopentenone is then protected (for subsequent reactions) with an appropriate blocking group $P_2$, as defined above.

The novel cyclopentenones (4a) are converted to the useful prostadienoates, most preferably, the compound of formula (1), by either of two routes.

In the first route the cyclopentenone alkynoate (4a) is subjected to partial catalytic hydrogenation to provide the cis-alkenoate (10)

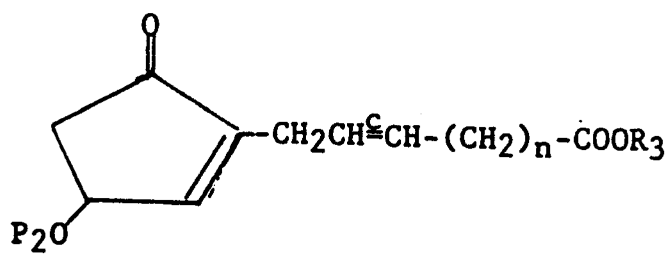

(10)

wherein $P_2$, n and $R_3$ are as defined hereinabove. This cyclopentenone (10), where n is 3 and $R_3$ is methyl, has been converted to the prostadienoate (1) by procedures described in J. Med. Chem., 25, 492 (1982).

In the second route, the cyclopentenone alkynoate (4a), where $P_1$ is $P_2$, is reacted with a lithio-cuprate reagent, or a functional equivalent thereof, which is capable of furnishing a prostaglandin beta-chain (11)

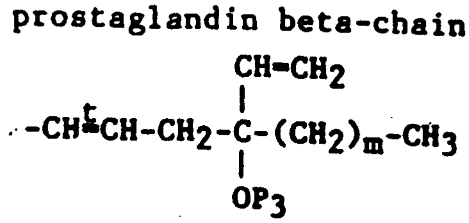

(11)

wherein m is an integer from 2-4 inclusive; and $P_3$ is a monovalent protective group with the same characteristics described above pertaining to $P_2$, and may be the same chemical moiety as, or may be different from, $P_2$.

A suitable lithio-cuprate reagent is disclosed in J. Med. Chem., 25, 492 (1982).

The product of the above reaction is a racemic and diastereomeric mixture of compounds (12)

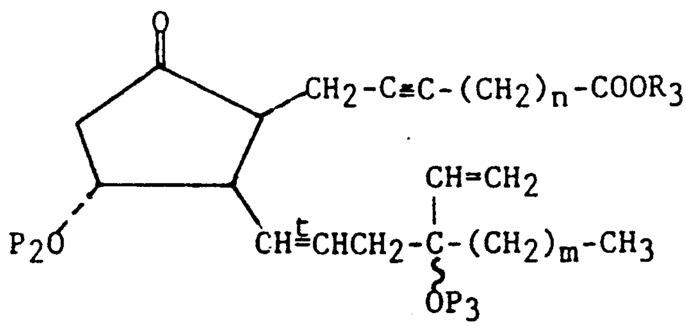

(12)

wherein m, n, $P_2$, $P_3$ and $R_3$ are as defined hereinabove.

The blocking groups $P_2$ and $P_3$ are then removed by hydrolysis to produce a mixture of diastereoisomers (13).

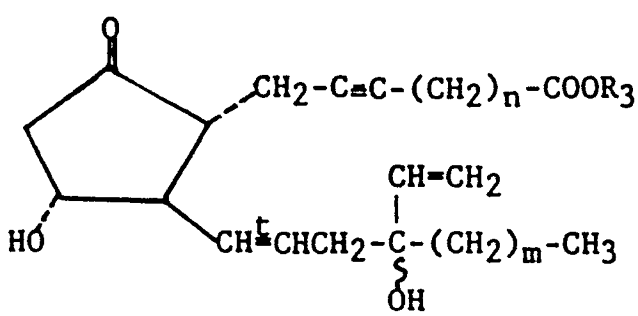

(13)

The triple bond of prostaglandin (13) is then subjected to partial catalytic hydrogenation to provide the prostadienoate (14).

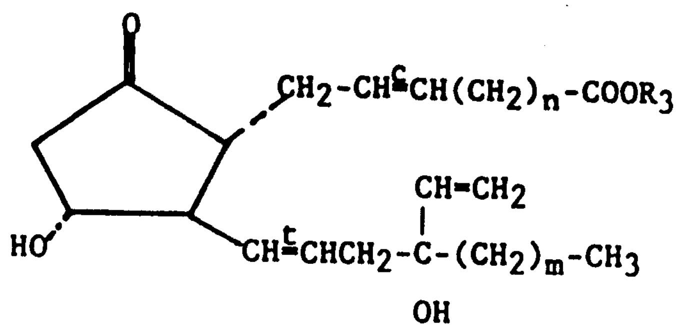

(14)

When n is 3, m is 3 and R3 is methyl, the product of the foregoing comprises a racemic mixture corresponding to formula (1), a pharmaceutical agent with the properties described above.

The dotted lines shown in the above formulae indicate that the substituents are in the alpha configuration, i.e. below the plane of the cyclopentenoyl ring.

Many of the compounds described herein possess asymmetric centers and thus can be produced as racemic mixtures or as individual enantiomers. The racemic mixtures can be resolved at appropriate stages by methods known to those skilled in the art, to obtain the respective individual enantiomers. It is to be understood that the racemic mixtures and the individual enantiomers are encompassed within the scope of this invention.

A major advantage of the novel processes and products of the invention is the production of pharmacologically active prostadienoates with greatly enhanced purity which is directly attributable to the partial catalytic hydrogenation of the triple bonds of the compounds of formulae (4a), in the first route, which is preferred, or of formulae (13), in the second route. NMR analysis shows that whereas the methods practiced heretofore resulted in a product containing about 8-11% impurities, the novel processes and products of the instant invention produce a product with an impurity level of only about 1-2%. The major contaminants in the previous

methods are compounds comprising the trans isomers of the pharmaceutically active prostadienoates, which are not the naturally-occurring biological form. By contrast, the partial catalytic hydrogenation of the triple bonds of the novel compounds of the invention is much more stereospecific, producing the biologically-occurring cis stereoisomers in significantly greater proportions without any additional purification procedures.

## DETAILED DESCRIPTION

In the most preferred embodiment of the novel processes of the invention, the novel compounds of this invention are prepared as described in Flowchart A, wherein $R_5$ is H and the substituents $R_1$, M, Y, n, $P_2$, $P_3$, and m are as described hereinabove.

0213313

## FLOWCHART A

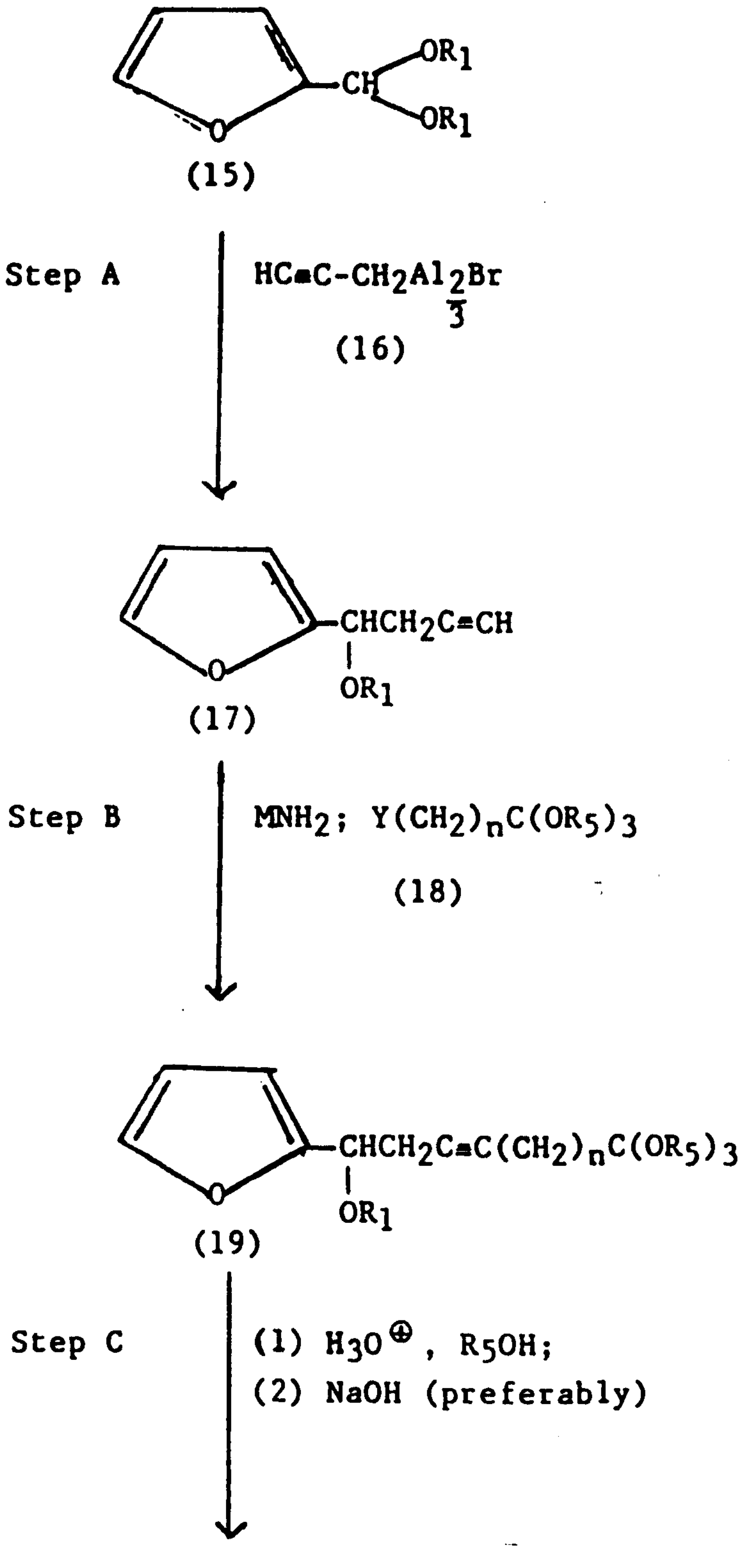


(15)

(16)

(17)

(18)

(19)

(20)

FLOWCHART A (continued)

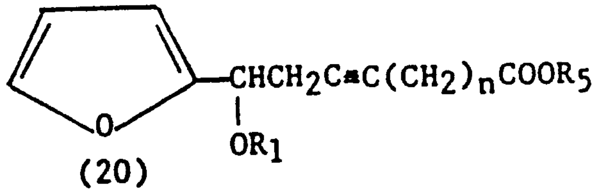

(20)

Step D

weak acid (e.g. phosphoric acid salt)

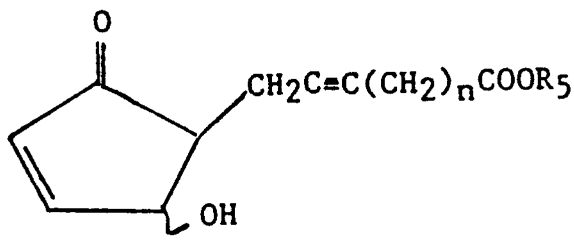

(21)

Step E

strong acid (e.g. sulfuric acid)

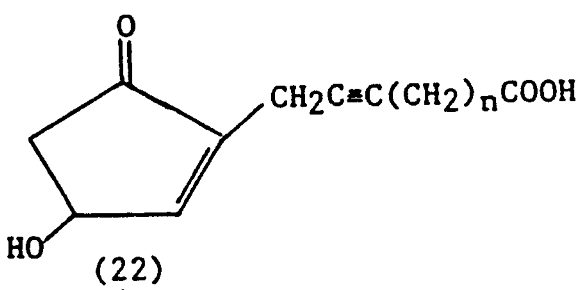

(22)

Step F

esterification

(23)

FLOWCHART A (continued)

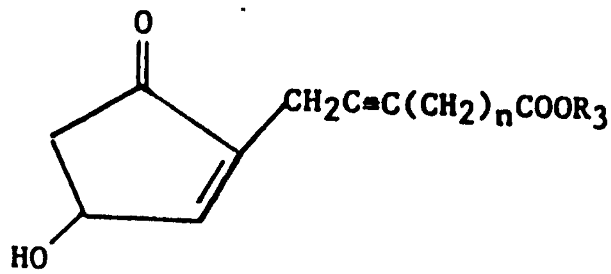

(23)

Step G

blocking the hydroxyl moiety

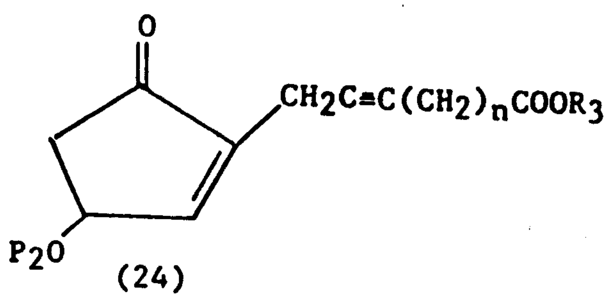

(24)

Step H

partial catalytic hydrogenation

(25)

## FLOWCHART A (continued)

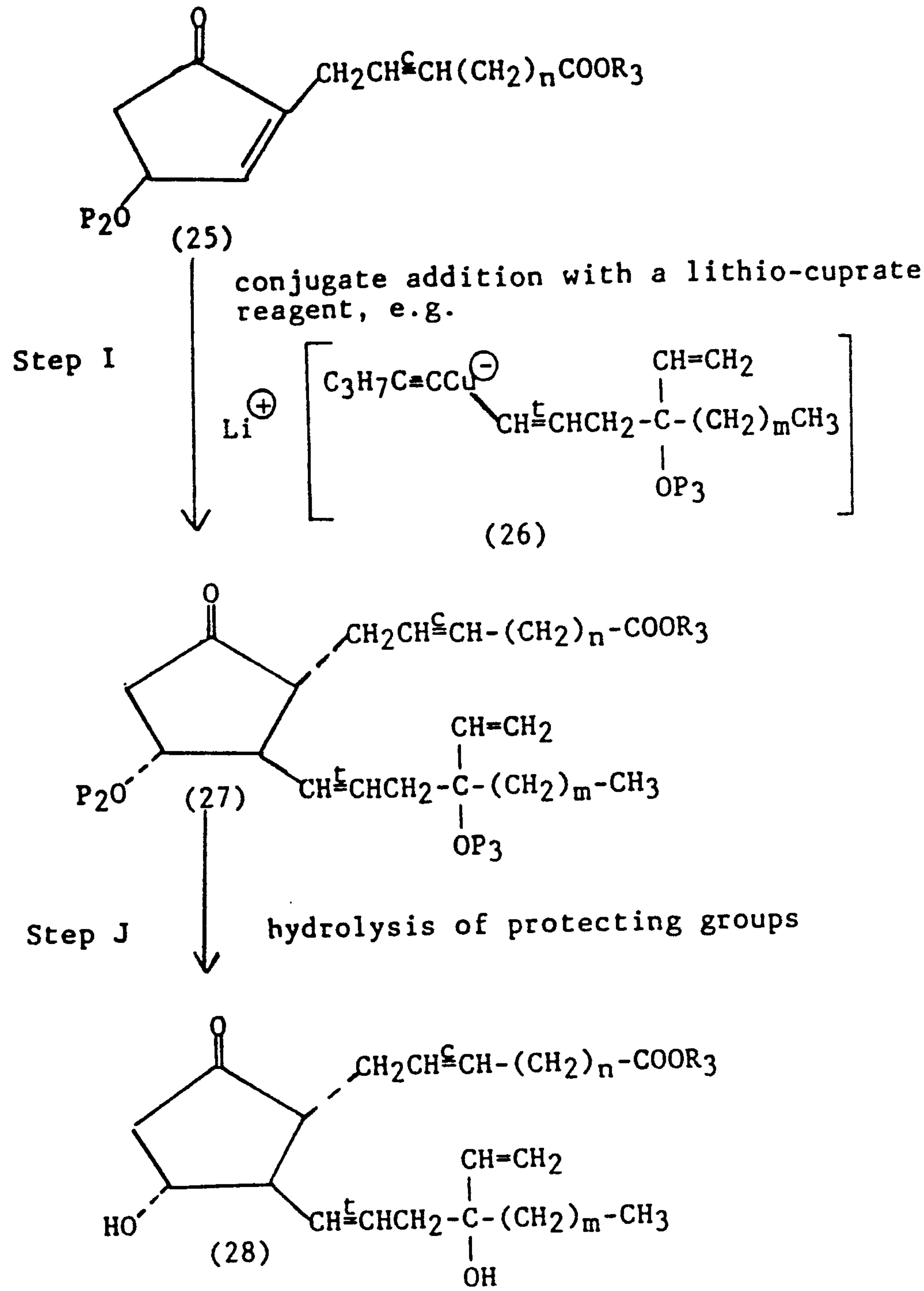


(25)

(26)

(27)

(28)

In accordance with the above flowchart A, a furfural dialkylacetal (15) is treated in step A with propargyl aluminum bromide (16) or its functional equivalent providing the ether (17). The metallation of the acetylene function in (17) is carried out preferably with an alkali metal amide in liquid ammonia. The preferred metal is lithium. The resulting species is not isolated but is reacted in step B with a bromide or iodide of formula (18) preferably in a cosolvent such as tetrahydrofuran and at -35° to +25°C. In the reactants of formula (18) the group $R_5$ in the orthoester function may be the same as or different from the group $R_3$ in formula (4a). The product of formula (19) is subjected in step C to mild acid hydrolysis in an alcohol solvent to provide the ester (20). The ester function is preferably hydrolyzed to the acid ($R_5$=H) under basic conditions, or optionally kept intact for the next stage, as shown in flowchart A.

The furylcarbinol ether (20) is then subjected in step D to a weak acid-catalyzed hydrolysis and rearrangement to a 3-hydroxycyclopentenone (21), usually present as a mixture with the 4-hydroxycyclopentenone isomer of (22). The reaction is accomplished with an aqueous or aqueous organic solution of a weak organic acid such as formic acid, or alternatively with a phosphate buffer solution having a pH of about 1-5. The most preferred conditions employ an aqueous solution of sodium dihydrogen phosphate at about 100°C. The isomerization of the 3-hydroxy isomer (21) to the 4-hydroxy isomer (22) is completed in step E by the addition of a strong acid, such as sulfuric acid, to the solution of isomers, preferably in the same reaction zone.

The resulting carboxylic acid (22) is then esterified in step F, for instance by alkylation with an alkyl halide ($R_3$-X) such as iodomethane, or by controlled Fisher esterification, for example, with methanol.

The hydroxyl moiety of compound (23) is provided in step G with a protective group $P_2$ capable of subsequent

removal under mild conditions, without effect on the rest of the molecule and capable of stability under the conditions of the partial catalytic hydrogenation or the conditions of the conjugate addition reaction. Advantageously, $P_2$ is trimethylsilyl or tetrahydropyranyl.

The partial catalytic hydrogenation, step H, of the triple bond in the compound (24) is preferably carried out with a poisoned palladium catalyst, or its equivalent, in a hydrocarbon solvent at -10° to +25°C under one atmosphere of hydrogen. The preferred catalyst is palladium on calcium carbonate, lead-poisoned (Lindlar catalyst).

The product cis-olefin (25) wherein $P_2$ is trimethylsilyl, n is 3 and $R_3$ is methyl, is a known intermediate for the preparation of the useful prostadienoate ester of formula (1). The requisite transformations which eventually provide the product (28) are depicted in steps I and J and are described in J. Med. Chem., 25, 492 (1982).

A major advantage of the above embodiment of the present invention, in addition to the high degree of stereoselection in the conversion of the acetylene group to a cis-olefin group through the agency of controlled partial catalytic hydrogenation is the increased ease of purification of the crystalline compound (23), where n is 3 and $R_3$ is hydrogen, and the concomitantly decreased cost of preparation.

An alternative method for the preparation of prostadienoate esters such as (1) is shown in flowchart B, wherein $P_2$, n, $R_3$, $P_3$ and m are as hereinabove described.

FLOWCHART B

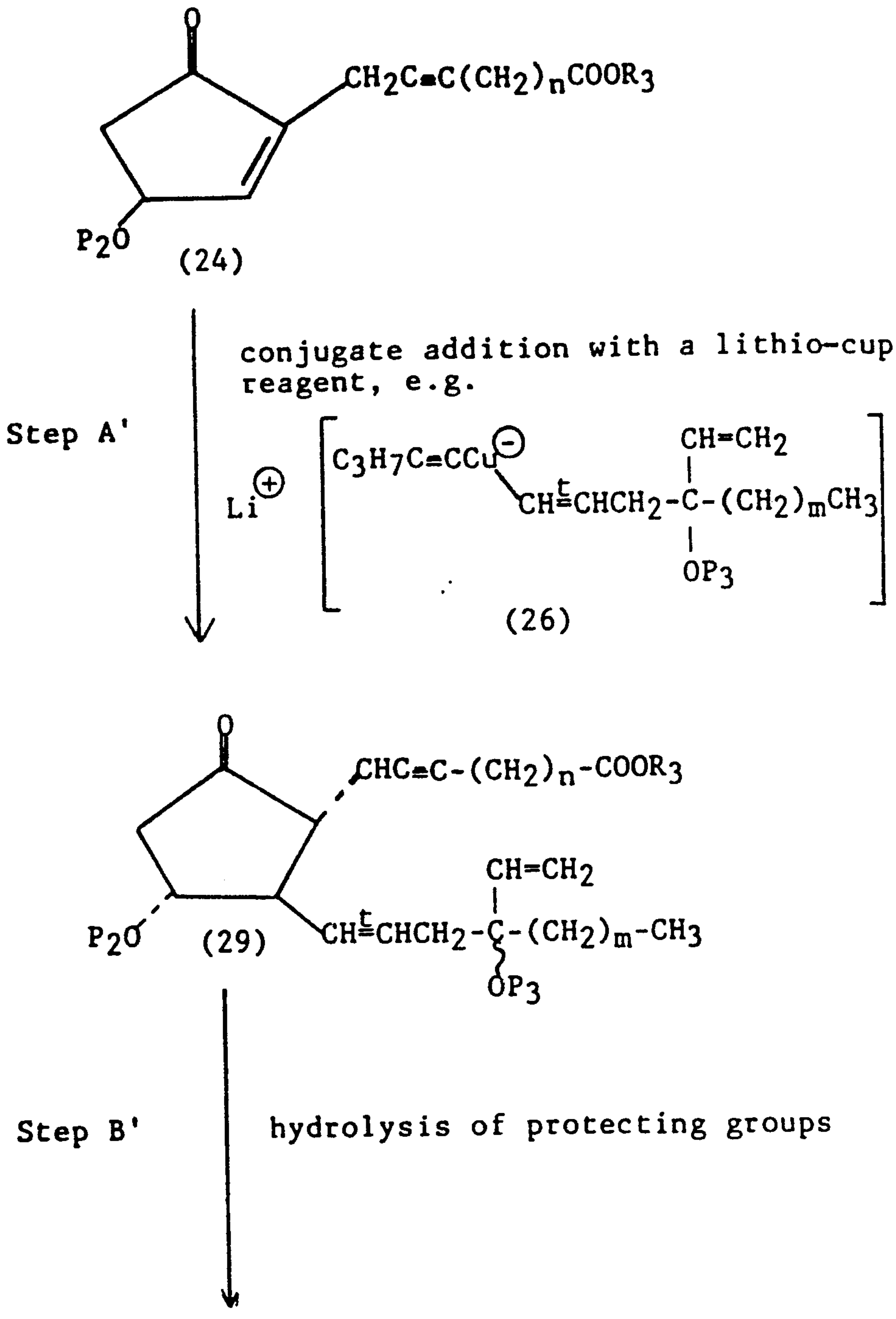


(24)

(26)

(29)

(30)

FLOW CHART B (continued)



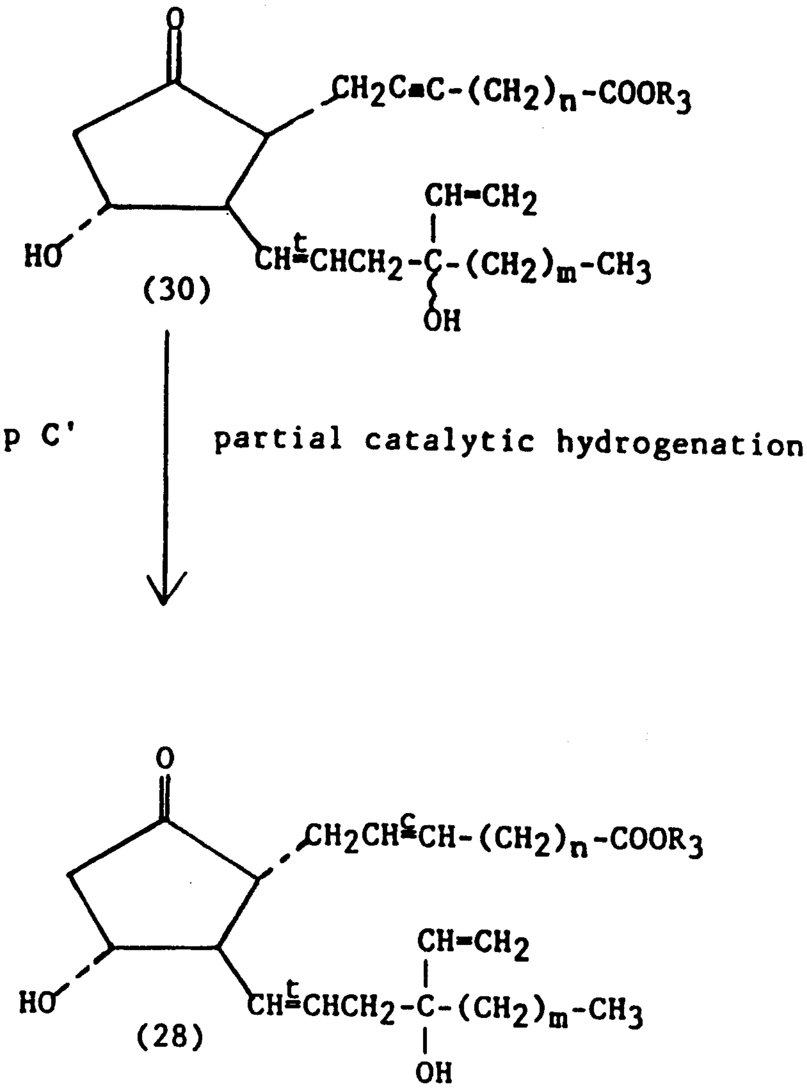


(30)

(28)

In accordance with flowchart B, the blocked cyclopentenone (24), prepared as described in flowchart A, is reacted in step A' with a lithio-cuprate reagent, preferably the reagent (26). The resulting blocked prostenynoate ester (29) is subjected to mild hydrolysis of the protecting groups $P_2$ and $P_3$ as shown in step B'. The preferred conditions employ acetic acid in aqueous tetrahydrofuran at 25-40°C. The resulting acetylenic diol (30) is subjected to partial catalytic hydrogenation in step C' to provide the useful prostadienoate esters (28). The preferred conditions employ a hydrocarbon solvent at 25-40°C under one atmosphere of hydrogen. A useful catalyst is palladium on calcium carbonate, lead-poisoned (Lindlar catalyst).

Also included in the scope of this invention are alternative processes for the preparation of novel intermediate cyclopentenone alkynoates as shown in flowchart C, wherein n and M are as described hereinabove and $R_6$ is $C_1$-$C_4$ alkyl.

**FLOWCHART C**



$HC \equiv C-(CH_2)_nCOOH$

(31)

Step A" $MR_6$

$MC \equiv C-(CH_2)_nCOOM$

(32)

Step B"

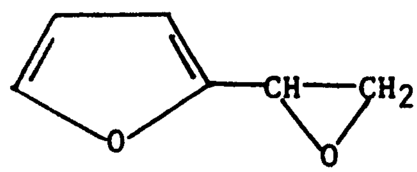

(33)

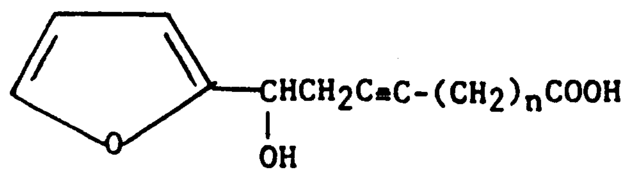

(34)

Step C" weak acid, e.g. phosphoric acid salt

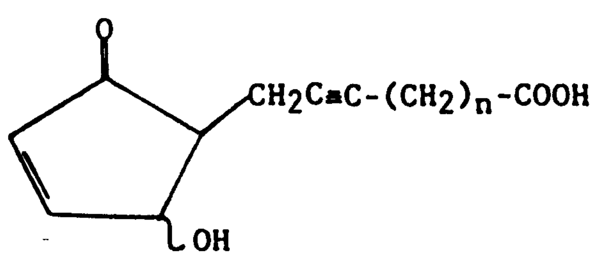

(35)

In accordance with flowchart C, treatment in step A" of an ω-carboxy-1-alkyne (31) with a strong alkali metal base, preferably an alkyllithium, in a suitable solvent provides a dianionic species (32). The preferred solvent for this reaction is hexamethylphosphoramide. The intermediate (32) is not isolated but is reacted in step B" with 2-(2-furyl)oxirane (33) to provide the hydroxy acid (34). The conversion of (34) to the cyclopentenone hydroxy acid isomer (35) is accomplished in step C" using the method described in detail above for step D of flowchart A.

An additional novel route to the cyclopentenone alkynoates of this invention is shown in the following flowchart D, wherein n is as hereinabove defined.

FLOWCHART D

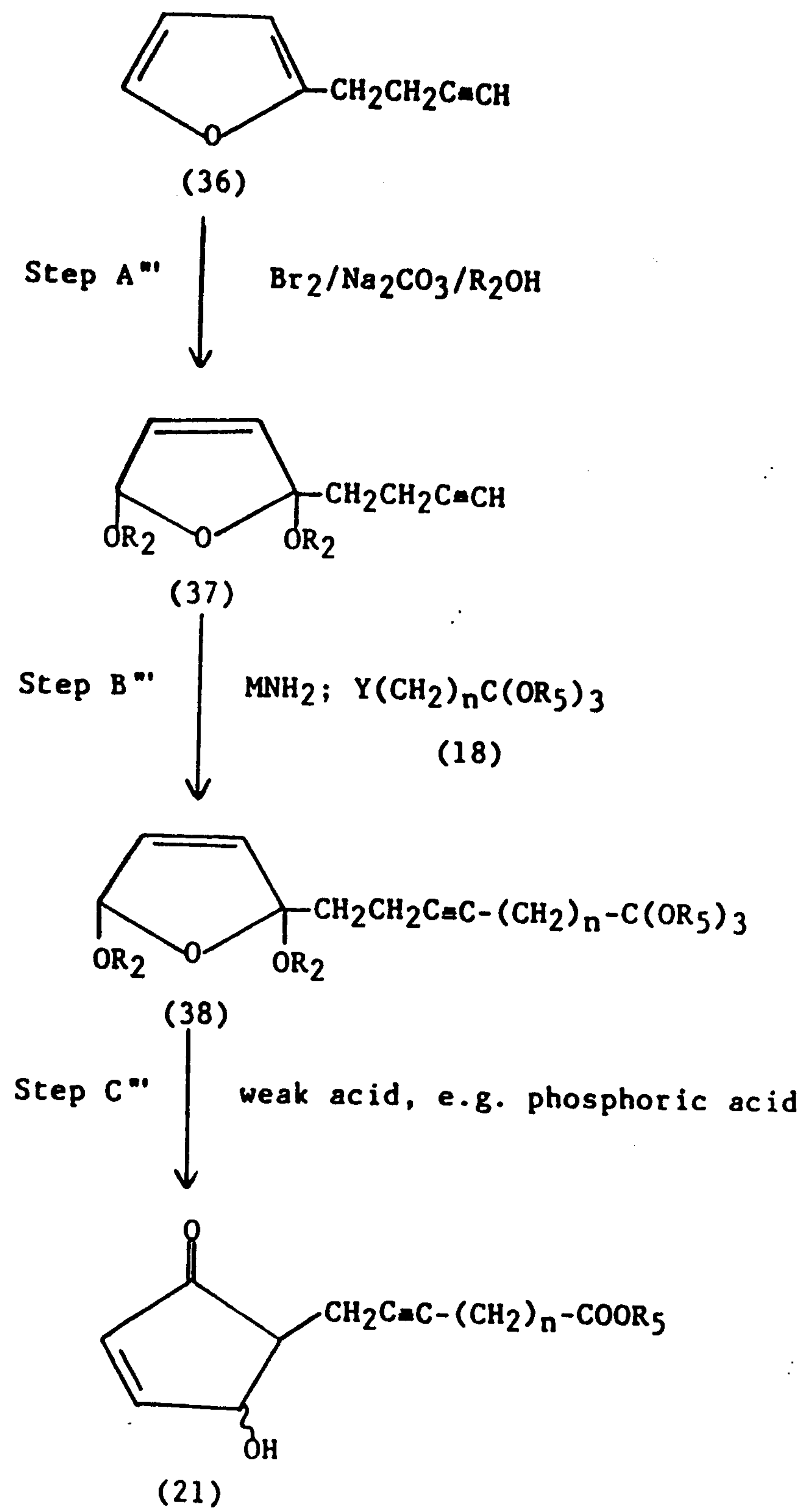

(36)
Step A'''
$Br_2/Na_2CO_3/R_2OH$
(37)
Step B'''
$MNH_2$; $Y(CH_2)_nC(OR_5)_3$
(18)
(38)
Step C'''
weak acid, e.g. phosphoric acid
(21)

In accordance with flowchart D, 4-(2-furyl)-1-butyne (36) is reacted in step A"' with bromine and sodium carbonate in a lower alcohol solvent, preferably methanol. The dialkoxydihydrofuran (37) is treated in step B"' with an alkali metal amide, preferably lithium amide, in liquid ammonia. The resulting acetylide salt is not isolated but is reacted with a bromide or iodide (18), preferably in a cosolvent such as tetrahydrofuran at -35 to +25°C. The dialkoxydihydrofuran (38) is then subjected in step C"' by a weak acid-catalyzed hydrolysis and rearrangement to the 3-hydroxycyclopentenone alkynoate (21). The group $R_5$ in the orthoester and ester functions of (38) and (21) respectively may be the same or different from the group $R_3$ present in (24) of flowchart A. The preferred conditions for the reaction of step C"' employ a phosphate buffer solution in an aqueous organic solvent having a pH of about 4-6. The conversion of the cyclopentenone (21) to the compound (28) is carried out as shown in flowchart A.

An additional novel route to the cyclopentenone alkynoates of this invention is shown in the following flowchart E, wherein n and y are as hereinabove defined.

In accordance with flowchart E, the ether (17) of flowchart A is reacted in Step A"" with an alkali metal amide followed by a chloroalkyl bromide or iodide of formula (39) in liquid ammonia with tetrahydrofuran cosolvent. The product chloride of formula (40) is reacted in step B with an alkali metal cyanide in a suitable solvent, preferably hexamethylphosphoramide, to provide the corresponding nitrile of formula (41). Hydrolysis of the nitrile is accomplished with an alkali metal hydroxide, preferably sodium hydroxide, in a suitable solvent, for example, water or aqueous ethanol. The conversion of the product acid (42) to the compound (28) is carried out as shown in flowchart A for compound (20), where $R_5$ is hydrogen.

FLOWCHART E



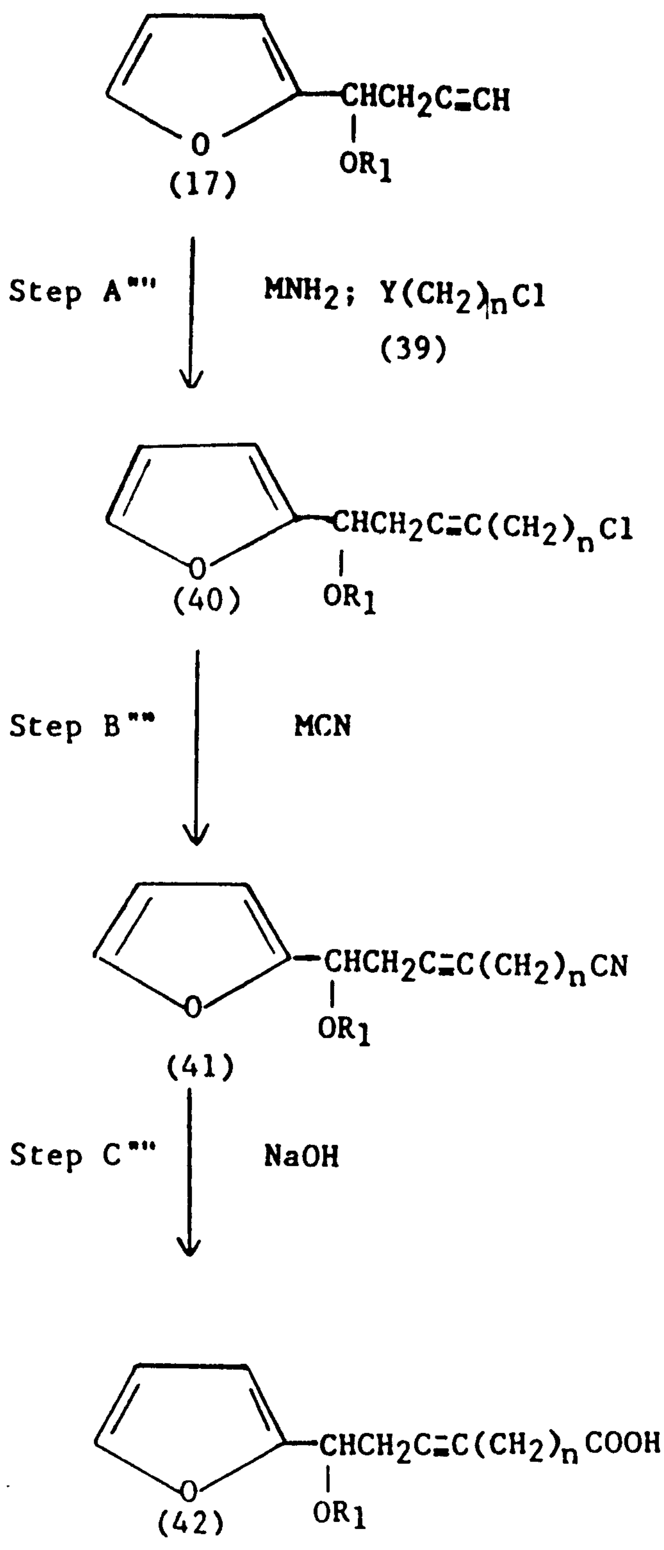

The invention will be described in greater detail in conjunction with the following examples.

Example 1

4-(2-Furyl)-4-methoxy-1-butyne

To a stirred solution of propargyl aluminum bromide, prepared from 52.9 g of 80% propargyl bromide in toluene, 6.41 g of aluminum splutters activated with 70 mg of mercuric choride and 80 ml of tetrahydrofuran, was added dropwise a solution of 42.2 g of furfural dimethyl acetal [J. Org. Chem., 40, 1478 (1975)] in 30 ml of tetrahydrofuran during a 15 minute period while maintaining a temperature of 25° to 30°C. The mixture was stirred for 20 minutes at 25°C, then partitioned with ether and ice-water. The organic layer was separated, washed with water, then aqueous sodium bicarbonate, finally with brine and dried. After evaporation the residue was distilled, giving 39.8 g of the desired compound, bp 68-69°C (11mm).

Example 2

Methyl 8-(2-furyl)-8-methoxy-5-octynoate

To a stirred solution of 4.09 g of lithium amide and 200 ml of liquid ammonia was added dropwise a solution of 24.3 g of 4-(2-furyl)-4-methoxy-1-butyne in 20 ml of tetrahydrofuran during 10 minutes. The mixture was stirred under reflux for 30 minutes and then treated dropwise with a solution of 40.4 g of 1-bromo-4,4,4-trimethoxybutane (U. S. Patent 3,864,387) in 40 ml of tetrahydrofuran during 10 minutes. This solution was stirred under reflux for 60 minutes, the ammonia was allowed to evaporate over one hour and the resulting solution stirred at 20°C for 10 minutes. The solution was then partitioned between ether and water, the organic layer separated, washed with brine, dried and concentrated, giving 58 g of crude 8-(2-furyl)-1,1,1,8-tetramethoxy-5-octyne as a liquid.

This liquid was dissolved in 160 ml of methanol and the solution was treated with 3.2 ml of 4N hydrochloric acid at 25°C. After 20 minutes the solution was treated with sodium bicarbonate solution and concentrated. The residue was extracted with ether. The extract was washed with brine, dried and concentrated. The residue was distilled at 0.08mm on a Kugelrohr at 110-120°C, giving 31.8 g of the desired compound as a colorless liquid.

Example 3

8-(2-Furyl)-8-methoxy-5-octynoic acid

A solution of 1.25 g of methyl 8-(2-furyl)-8-methoxy-5-octynoate, 0.66 g of 85% potassium hydroxide, 15 ml of methanol and 10 ml of water was heated under reflux for one hour. The methanol was evaporated and the residue partitioned with ether and water. The aqueous layer was chilled at 0° to 10°C, acidified with hydrochloric acid and the product extracted with ether. The extract was washed with brine, dried and concentrated, giving 1.13 g of the desired compound as a colorless oil. The identification is established by PMR; δ 3.35 (3H, s, methyl ether).

Example 4

Methyl 8-(2-Furyl)-8-hydroxy-5-octynoate

To a stirred solution of 16.3 g of 4-(2-furyl)-4-hydroxy-1-butyne [Ann. Chem. 682, 62 (1965)] in 120 ml of tetrahydrofuran was added 150 ml of a 1.6M solution of n-butyllithium in hexane, during 30 minutes at 0° to 10°C. After 15 minutes at 0° to 10°C, the stirred solution was treated dropwise with a solution of 30 g of 1-bromo-4,4,4-trimethoxybutane in 90 ml of hexamethylphosphoric triamide during 20 minutes while maintaining a temperature of -10° to 0°C. The mixture was warmed to 25°C, stirred for 18 hours, then poured into ice water and the crude product extracted with ether.

The extract, which contained 8-(2-furyl)-8-hydroxy-1,1,1-trimethoxy-5-octyne, was thoroughly washed with water and then stirred vigorously with 150 ml of 0.25N hydrochloric acid at 10° to 15°C for 15 minutes. The mixture was then treated with 100 ml of saturated sodium bicarbonate and the organic layer separated, washed with brine, dried and concentrated. The residue was distilled at 0.09mm on a Kugelrohr at 145-150°C, giving 15.8 g of the desired compound as an oil.

Example 5

8-(2-Furyl)-8-methoxy-5-octynoic acid

To a stirred solution of 1.25 g of methyl 8-(2-furyl)-8-methoxy-5-octynoate in 15 ml of methanol was added 10 ml of 1N potassium hydroxide. This mixture was stirred at reflux for one hour, then cooled and concentrated. The residue was partitioned between water and ether. The aqueous layer was separated, cooled to 0°C and acidified with 1N hydrochloric acid, then saturated with sodium chloride and extracted with ether. The ether extract was washed with brine, dried and concentrated, giving 1.13 g of the desired compound as an oil; PMR δ 4.38 (1H, t, C<u>H</u>OMe).

Example 6

7-(4-Hydroxycyclopent-2-en-1-on-2-yl)-5-heptynoic acid

A stirred solution of 25 g of 8-(2-furyl)-8-methoxy-5-octynoic acid, 73 g of sodium dihydrogen phosphate monohydrate, and 2.12 l of water was heated under reflux for 72 hours. The resulting solution, which contained a mixture of the 3- and 4- hydroxy isomers, was cooled to 50° and treated dropwise with 29.6 ml of concentrated sulfuric acid. The resulting mixture was stirred under reflux for 24 hours, cooled and filtered through celite. The filtrate was saturated with sodium chloride and extracted with ethyl acetate. The extract was washed with brine, dried and concentrated to give a solid. Recrystallization from ether gave 15.2 g of the desired product, mp 68-74°.

Example 7

2,5-Dihydro-2,5-dimethoxy-2-(3-butynyl)furan

To a stirred mixture of 6.01 g of 4-(2-furyl)-1-butyne [Perfumery Essent. Oil Record., 57, 364 (1966)], 10.6 g of sodium carbonate and 150 ml of methanol at -25°C, was added dropwise a solution of 8.0 g of bromine in 50 ml of methanol during a period of 3 hours. The mixture was then stirred at 25°C for 45 minutes and concentrated *in vacuo*. The residue was partitioned with ether and brine. The ether layer was separated, washed with brine, dried and concentrated. Distillation of the residue at 0.5mm gave 6.62 g of the desired compound as a light yellow liquid, bp 57-61°C (0.5mm).

Example 8

2,5-Dihydro-2,5-dimethoxy-2-(8,8,8-trimethoxy-3-octynyl)furan

To a stirred mixture of 0.51 g of lithium amide and 25 ml of liquid ammonia was added dropwise a solution of 3.64 g of 2,5-dihydro-2,5-dimethoxy-2-(3-butynyl)furan in 2.5 ml of tetrahydrofuran during 5 minutes. The mixture was stirred under reflux for 30 minutes and then treated dropwise with a solution of 5.0 g of 1-bromo-4,4,4-trimethoxybutane (U. S. Patent 3,864,387) in 5 ml of tetrahydrofuran during 5 minutes. This solution was stirred under reflux for one hour, the ammonia allowed to evaporate during one hour and the resulting solution stirred at 20°C for 10 minutes. This solution was partitioned with ether and brine. The organic layer was dried, treated with 2 drops of pyridine, and then concentrated, giving 7.1 g of the desired compound as a light amber oil; PMR $\delta$ 5.9 (2H, m, vinyl hydrogens).

Example 9

Trimethylsilyl 7-(4-trimethylsiloxycyclopent-2-en-1-on-2-yl)-5-heptynoate

A stirred mixture of 7.1 g of 2,5-dihydro-2,5-dimethoxy-2-(8,8,8-trimethoxy-3-octynyl)furan, 2.76 g of sodium dihydrogen phosphate monohydrate, 140 mg of disodium hydrogen phosphate, 60 ml of dioxane and 40 ml of water was

boiled under reflux for 19 hours, then cooled to 50°C and treated dropwise with 4.1 ml of concentrated sulfuric acid. The resulting solution was boiled under reflux for 12 hours, cooled and partitioned with ether and brine. The ether layer was separated, washed with brine, dried and concentrated, giving 5.0 g of crude 7-(4-hydroxycyclopent-2-en-1-on-2-yl)-5-heptynoic acid. This crude product was dissolved in 40 ml of pyridine and the stirred solution treated at 0° to 10°C with 10.5 ml of hexamethyldisilizane followed by 6.3 ml of chlorotrimethylsilane. The resulting mixture was stirred at 25°C for 3 hours and then concentrated to dryness at 30°C. The residue was extracted with hexane, the extract filtered through diatomaceous earth and concentrated. The residue was distilled at 0.11mm on a Kugelrohr at 155-170°C, giving 1.66 g of the desired compound as an oil.

## Example 10

## Methyl 7-(4-hydroxycyclopent-2-en-1-on-2-yl)-5-heptynoate

A stirred mixture of 5.8 g of 7-(4-hydroxycyclopent-2-en-1-on-2-yl)-5-heptynoic acid, 3.95 g of potassium carbonate, 7.4 g of iodomethane and 52 ml of acetone was refluxed for 2.5 hours. Most of the acetone was evaporated and the residue was partitioned with water and ether. The ether layer was separated, washed with brine, dried and concentrated, giving 5.02 g of the desired compound as an oil; PMR δ 3.72 (3H, s, methyl ester).

## Example 11

## Methyl 7-(4-trimethylsiloxycyclopent-2-en-1-on-2-yl)-5-heptynoate

To a stirred solution of 5.0 g of methyl 7-(4-hydroxycyclopent-2-en-1-on-2-yl)-5-heptynoate in 27 ml of pyridine was added successively 2.53 ml of hexamethyldisilizane and 0.76 ml of chlorotrimethylsilane while maintaining a temperature of 0° to 10°C. The mixture was then stirred at 25°C for 2 hours and then all volatile materials were evaporated at 30°C *in vacuo*. The residue was slurried with hexane and this solution was filtered through diatomaceous earth. The filtrate was evaporated and the residue distilled

at 0.06mm on a Kugelrohr, giving 4.79 g of the desired compound as a colorless oil, bp 135-140°C.

Example 12

Methyl 7-(4-trimethylsiloxycyclopent-2-en-1-on-2-yl)-5-cis-heptenoate

A stirred solution of 1.54 g of methyl 7-(4-trimethylsiloxycyclopent-2-en-1-on-2-yl)-5-heptynoate, 0.25 ml of synthetic quinoline, 7.5 ml of hexane and 2.5 ml of toluene was hydrogenated at 0°C and atmospheric pressure in the presence of 50 mg of lead-poisoned palladium on calcium carbonate (Lindlar catalyst) until hydrogen uptake nearly ceased (2.5 hours). The catalyst was removed by filtration and the filtrate was concentrated. The residue was distilled at 0.06mm on a Kugelrohr, giving 1.58 g of the desired compound as a colorless oil, bp 130-135°C.

Example 13

Methyl 9-oxo-11α,16-dihydroxy-16-vinylprosta-5-yn-13-trans-enoate

To a stirred solution of 6.44 g of 4-trimethylsiloxy-4-vinyl-1-(tri-n-butylstannyl)-trans-1-octane [J. Med. Chem., 25, 492 (1982)] in 10 ml of tetrahydrofuran at -78°C was added 6.25 ml of 1.6M n-butyllithium in hexane during 5 minutes. The resulting solution was stirred at -40°C for 2 hours. The stirred solution was recooled to -78°C and a solution prepared from 1.57 g of copper pentyne, 4.36 ml of hexamethylphosphorous triamide and 25 ml of ether was added. This solution was stirred at -78°C for one hour and then treated during 5 minutes with a solution of 1.54 g of methyl 7-(4-trimethylsiloxycyclopent-2-en-1-on-2-yl)-5-heptynoate in 5 ml of ether. This solution was stirred at -40°C for one hour, then at -20°C for 5 minutes, then recooled to -78°C and quenched with a solution of 1.2 ml of acetic acid in 10 ml of ether. This mixture was poured into a stirred mixture of ammonium chloride, dilute hydrochloric acid and ether. The ether phase was separated and washed successively with dilute hydrochloric acid, dilute brine-saturated sodium bicarbonate and finally brine. The washed solution was dried and concentrated, giving 8.2 g of light yellow oil. This oil was stirred with a solution of 40 ml of acetic acid, 20 ml of

tetrahydrofuran and 10 ml of water at 25°C for one hour. The solvents were evaporated with the aid of toluene and the residue partitioned with heptane and methanol. The methanol phase was concentrated and the residue purified by dry column chromatography on silica gel with 3:1 ethyl acetate:heptane, giving the desired compound as an oil; PMR δ 3.70 (3H, s, methyl ester) and 4.13 (1H, q, C<u>H</u>OH).

Example 14

Methyl 9-oxo-11α,16-dihydroxy-16-vinylprosta-5-*cis*-13-*trans*-dienoate

A stirred solution of 0.86 g of methyl 9-oxo-11 , 16-dihydroxy-16-vinylprosta-5-yn-13-*cis*-enoate in 2.2 ml of hexane and 2.2 ml of toluene was treated with 0.11 ml of synthetic quinoline and 22 mg of palladium on calcium carbonate catalyst (lead-poisoned; Lindlar catalyst) under argon. Hydrogen gas at one atmosphere was introduced and the mixture was stirred at 40°C until 50 ml of hydrogen had been absorbed. The catalyst was filtered off and the filtrate concentrated. The residue was subjected to dry column chromatography on silica gel with 3:1 ethyl acetate:heptane, giving the desired compound as an oil; PMR δ 5.55 (2H, m, *cis*-CH=CH).

Examples 15-17

Following the general procedure of Example 6, treatment of the furans in the following Table with sodium dihydrogen phosphate followed by sulfuric acid provides the cyclopentenolone product of Example 6.

TABLE

| Example | Starting Material |
|---|---|
| 15 | 8-(2-furyl)-8-methoxy-5-octynoic acid |
| 16 | methyl 8-(2-furyl)-8-hydroxy-5-octynoate |
| 17 | 8-(2-furyl)-8-hydroxy-5-octynoic acid |

Example 18

8-(2-Furyl)-8-hydroxy-5-octynoic acid

To a stirred solution of 2.80 g of 5-pentynoic acid [Chem. Abst., 59, 9782d (1963)] in 50 ml of hexamethylphosphorous triamide was added 31.2 ml of 1.6M *n*-butyllithium in hexane during 20 minutes at 15°C. After the

addition, the solution was stirred for 10 minutes, then cooled to 0°C and treated with 2.75 g of 2-(2-furyl)oxirane [Zh. Organ. Khim., 1, 539 (1965)]. The solution was warmed to 25°C, stirred for 5 hours and then partitioned with ether and dilute hydrochloric acid at 0°C. The ether layer was separated, washed, dried and evaporated. The resulting oil was chromatographed on silica gel, giving 2.49 g of the desired compound as an oil; PMR δ 4.85 (1H, t, CHOH).

Example 19

7-Chloro-1-(2-furyl)-1-methoxy-3-heptyne

To a stirred mixture of 0.51 g of lithium amide and 25 ml of liquid ammonia was added a solution of 3.0 g of 4-(2-furyl)-4-methoxy-1-butyne in 2.5 ml of tetrahydrofuran. The mixture was stirred under reflux for 30 minutes and then treated with a solution of 3.94 g of 1-bromo-3-chloropropane in 5 ml of tetrahydrofuran. This mixture was stirred under reflux for one hour, then the ammonia was allowed to evaporate and the residue partitioned with ether and water. The ether layer was separated and evaporated. The residue was distilled at 0.06mm on a Kugelrohr, giving 3.07 g of the desired compound as an oil, bp 110-120°C.

Example 20

7-Cyano-1-(2-furyl)-1-methoxy-3-heptyne

A stirred mixture of 0.45 g of 7-chloro-1-(2-furyl)-1-methoxy-3-heptyne, 0.20 g of sodium cyanide, and 4.0 ml of hexamethylphosphoric triamide was maintained at 25°C for 22 hours. The mixture was partitioned with water and 1:1 hexane-ether. The organic layer was washed with water and brine, dried and concentrated. The resulting oil was distilled at 0.30 mm on a Kugelrohr at 105-110°C, giving 0.38 g of the desired compound as an oil; PMR δ 2.36 (2H, t, $CH_2CN$).

Example 21

8-(2-Furyl)-8-methoxy-5-octynoic acid

A stirred mixture of 0.22 g of 7-cyano-1-(2-furyl)-1-methoxy-3-heptyne and 5.0 ml of 2.5 N sodium hydroxide was refluxed for 5 hours. The resulting solution was cooled, extracted with ether, and acidified at 0° with hydrochloric

acid. The resulting mixture was extracted with ether. The extract was washed with brine, dried and concentrated to give the desired acid as an oil; PMR δ 3.35 (3H, s, methylether) and 2.40 (2H, t, $C\underline{H}_2COOH$).

CLAIMS

WHAT IS CLAIMED IS:

1. A compound of the formula

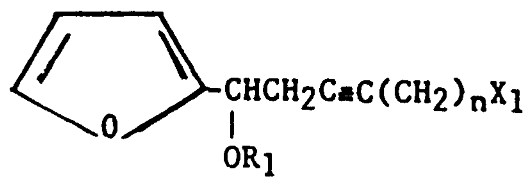

wherein $R_1$ is selected from the group consisting of hydrogen and $C_1$-$C_4$ alkyl; $X_1$ is selected from the group consisting of halogen, cyano, $C_1$-$C_4$ alkoxycarbonyl, carboxy and tri-($C_1$-$C_4$ alkoxy)methyl; and n is an integer 2-4 inclusive.

2. A compound of the formula

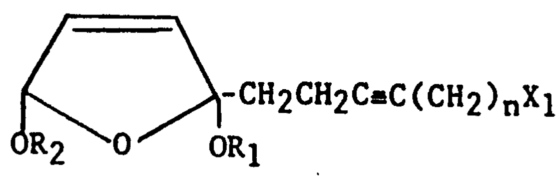

wherein $R_2$ is $C_1$-$C_4$ alkyl; $X_1$ is selected from the group consisting of halogen, cyano, $C_1$-$C_4$ alkoxycarbonyl, carboxy and tri-($C_1$-$C_4$ alkoxy)methyl; and n is an integer 2-4 inclusive.

3. A compound of the formula

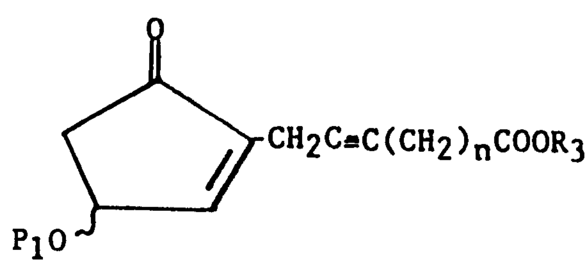

or

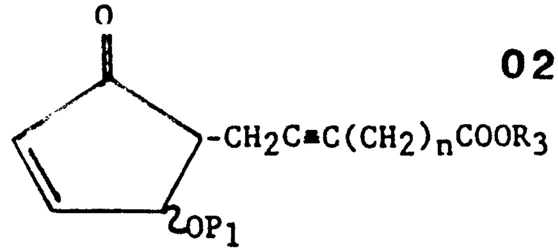

wherein $R_3$ is a monovalent radical selected from the group consisting of hydrogen, $C_1$-$C_4$ trialkylsilyl and $C_1$-$C_6$ alkyl; n is an integer 2-4 inclusive; $P_1$ is a monovalent radical selected from the group consisting of hydrogen and $P_2$; and $P_2$ is a blocking or protective group selected from the group consisting of tetrahydropyranyl, trialkylsilyl and alpha-alkoxyethyl.

4. A process for preparing compounds of the formula

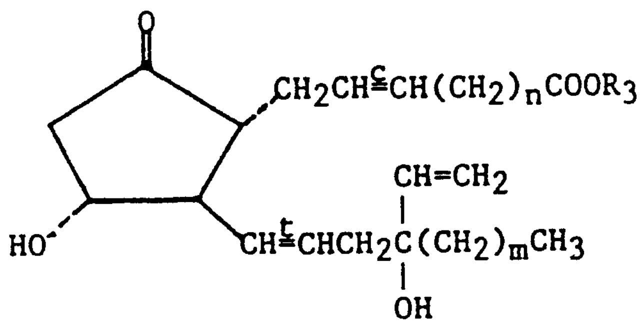

wherein n and m represent integers 2-4 inclusive and $R_3$ is a monovalent radical selected from the group consisting of hydrogen, $C_1$-$C_4$ trialkylsilyl and $C_1$-$C_6$ alkyl, which comprises the steps of:

(a) treating a furfuryl dialkylacetal of the formula

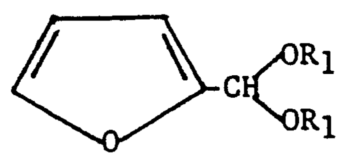

where $R_1$ is selected from the group consisting of hydrogen and $C_1$-$C_4$ alkyl, with propargyl aluminum bromide, giving a compound of the formula

0213313

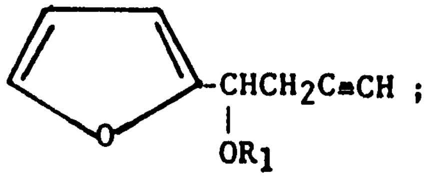

(b) reacting the compound produced in step (a) with an alkali metal amide in liquid ammonia and then with a compound of the formula $Y(CH_2)_nC(OR_5)_3$, where $R_5$ is $C_1$-$C_4$ trialkylsilyl or $C_1$-$C_6$ alkyl and Y is bromine or iodine, giving a compound of the formula

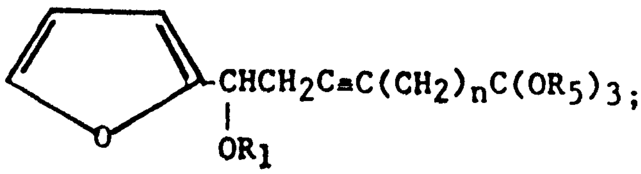

(c) subjecting the compound produced in step (b) to mild acid hydrolysis in an alcohol solvent $R_5OH$, giving an ester of the formula

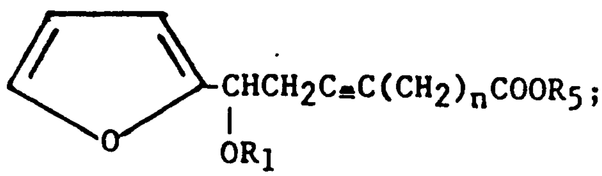

(d) subjecting the ester produced in step (c) to weak acid-catalyzed hydrolysis and rearrangement giving a 3-hydroxycyclopentenone of the formula

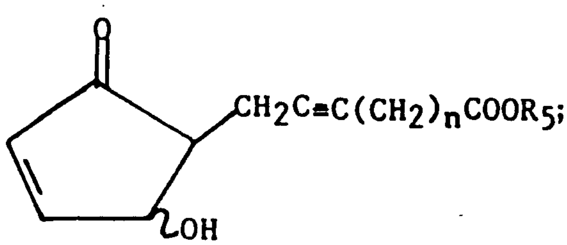

(e) isomerizing the 3-hydroxycyclopentenone produced by step (d) to produce the 4-hydroxycyclopentenone carboxylic acid of the formula

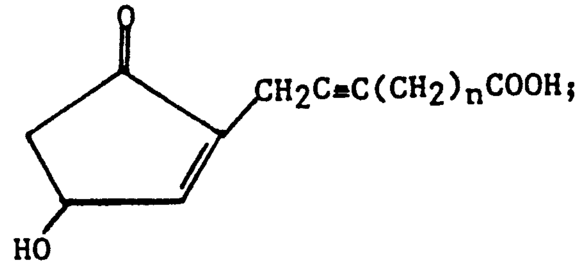

(f) esterifying the carboxylic acid produced in step (e) to produce a compound of the formula

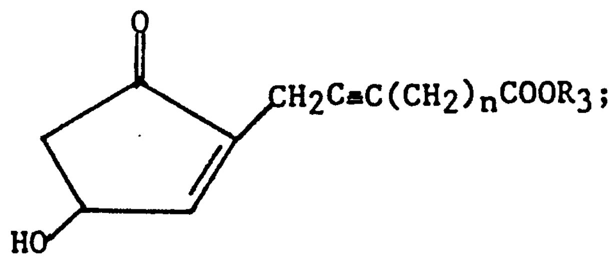

(g) protecting the hydroxy group of the compound produced by step (f) with a blocking group $P_2$ selected from the group consisting of tetrahydropyranyl, trialkylsilyl and alphaalkoxyethyl, giving a compound of the formula

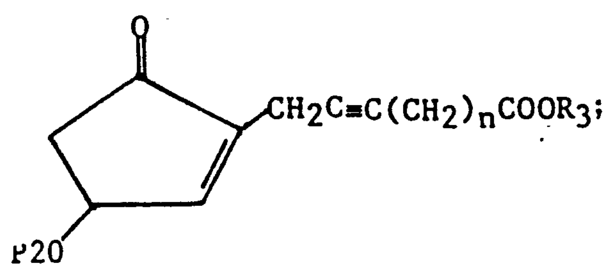

and

(h) subjecting the protected compound produced in step (g) to partial catalytic hydrogenation giving a compound of the formula

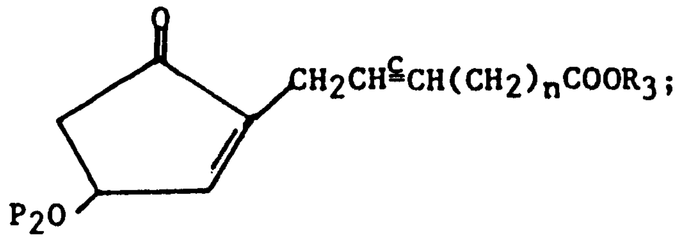

; and

(i) subjecting the compound produced step (h) to conjugate addition and hydrolysis to produce the desired compounds.

5. A process for preparing compounds of the formula

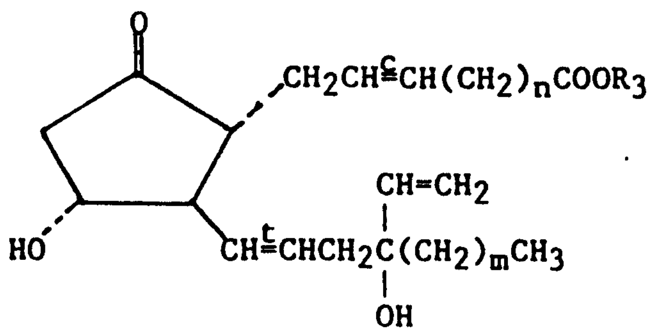

wherein n and m are integers 2-4 inclusive and $R_3$ is a monovalent radical selected from the group consisting of hydrogen, $C_1$-$C_4$ trialkylsilyl and $C_1$-$C_6$ alkyl, which comprises the steps of:

(a) reacting a cyclopentenone of the formula

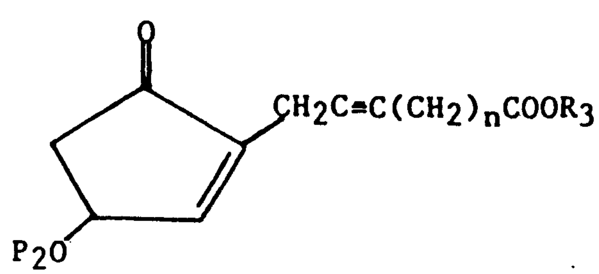

,

where $P_2$ is a protecting agent selected from the group consisting of tetrahydropyranyl, trialkylsilyl and alpha-alkoxyethyl with a lithio-cuprate reagent of the formula

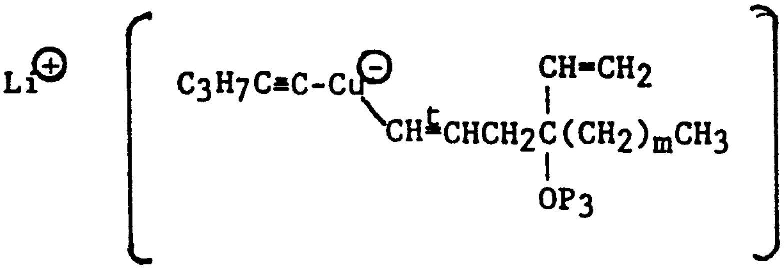

, where $P_3$ is selected from the same group as $P_2$, giving a compound of the formula

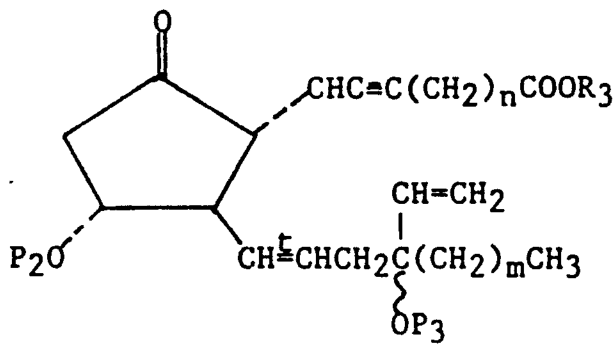

;

(b) subjecting the compound produced in step (a) to mild hydrolysis producing an acetylenic diol of the formula

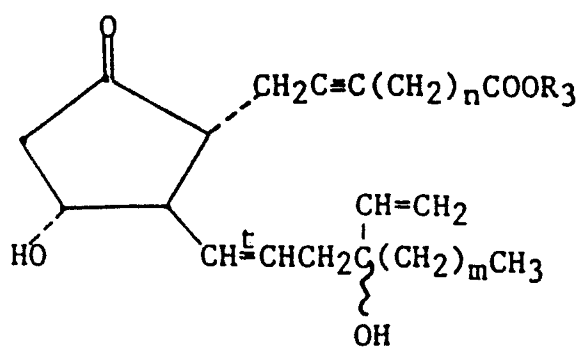

; and

(c) subjecting the acetylenic diol produced in step (b) to partial catalytic hydrogenation to produce the desired products.

6. A process of producing cyclopentenone alkynoates of the formula

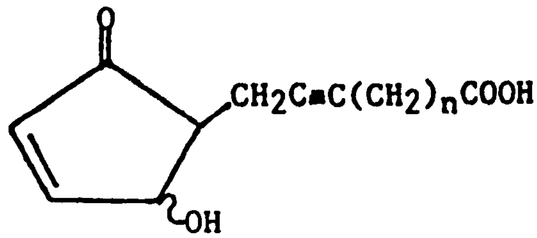

where n is an integer 2-4 inclusive, which comprises the steps of:

(a) reacting a compound of the formula $HC\equiv C(CH_2)_nCOOH$ with the compound $MR_6$, wherein $R_6$ is $C_1$-$C_4$ alkyl and M is lithium, sodium or potassium, giving compounds of the formula $MC\equiv C(CH_2)_nCOOM$;

(b) treating the product of step (a) with 2-(2-furyl)oxirane providing a hydroxy acid of the formula

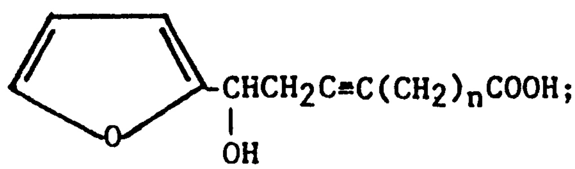

; and

(c) subjecting the hydroxy acid produced in step (b) to a weak acid-catalyzed hydrolysis and rearrangement to produce the desired products.

7. A process for producing cyclopentenone alkynoates of the formula

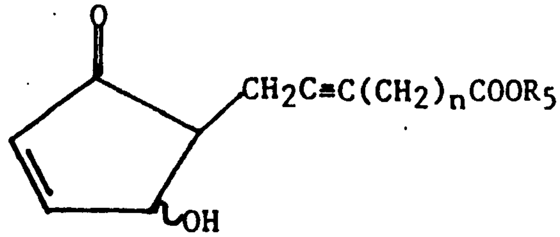

,

wherein n is an integer 2-4 inclusive and $R_5$ is a monovalent radical selected from the group consisting of hydrogen, $C_1$-$C_4$ trialkylsilyl and $C_1$-$C_6$ alkyl, which comprises the steps of:

(a) reacting 4-(2-furyl)-1-butyne with bromine and sodium bicarbonate in a solvent of the formula $R_2OH$, where $R_2$ is $C_1$-$C_4$ alkyl, giving a dialkoxydihydrofuran of the formula

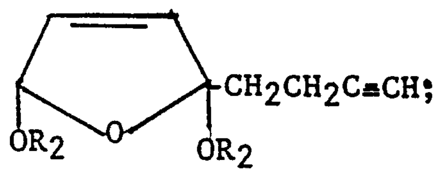

(b) reacting the dialkoxydihydrofuran produced in step (a) with $MNH_2$, wherein Mi is lithium, sodium or potassium;

(c) reacting the compound produced in step (b) with a compound of the formula $Y(CH_2)_nC(OR_5)_3$, where Y is bromine or iodine, to produce a dialkoxydihydrofuran of the formula

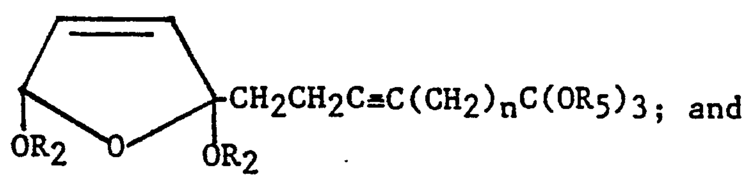

(d) subjecting the dialkoxydihydrofuran produced in step (c) to a weak acid-catalyzed hydrolysis and rearrangement to produce the desired products.

8. A process for preparing compounds of the formula

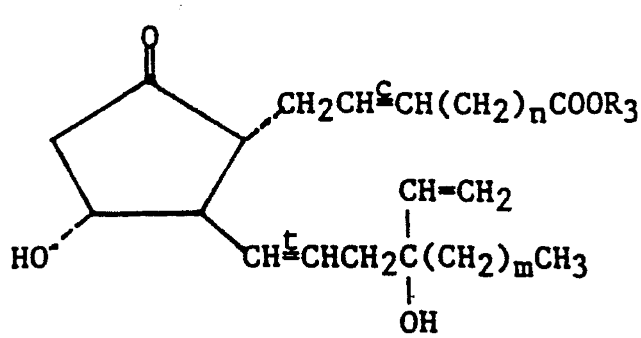

wherein n and m represent integers 2-4 inclusive and $R_3$ is a monovalent radical selected from the group consisting of hydrogen, $C_1$-$C_4$ trialkylsilyl and $C_1$-$C_6$ alkyl, which comprises the steps of:

(a) treating a furfuryl dialkylacetal of the formula

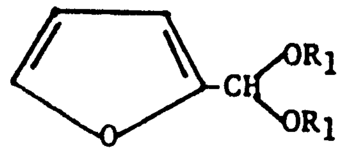

where $R_1$ is selected from the group consisting of hydrogen and $C_1$-$C_4$ alkyl, with propargyl aluminum bromide, giving a compound of the formula

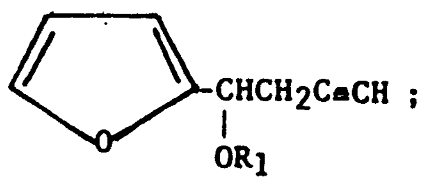

(b) reacting the compound produced in step (a) with an alkali metal amide in liquid ammonia and then with a compound of the formula $Y(CH_2)_nC(OR_5)_3$, where $R_5$ is $C_1$-$C_4$ trialkylsilyl or $C_1$-$C_6$ alkyl and Y is bromine or iodine, giving a compound of the formula

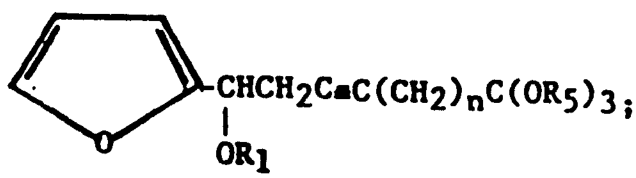

(c) subjecting the compound produced in step (b) to mild acid hydrolysis in an alcohol solvent $R_5OH$, giving an ester of the formula

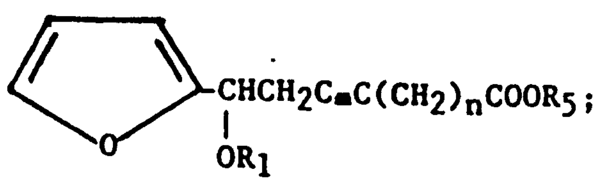

(d) subjecting the ester produced in step (c) to saponification, giving an acid of the formula

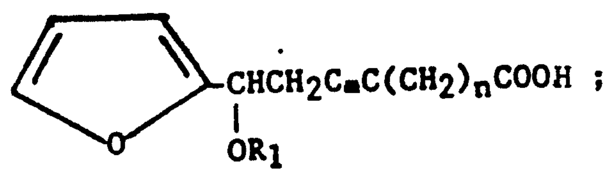

(e) subjecting the acid produced in step (d) to weak acid-catalyzed hydrolysis and rearrangement giving a 3-hydroxycyclopentenone of the formula

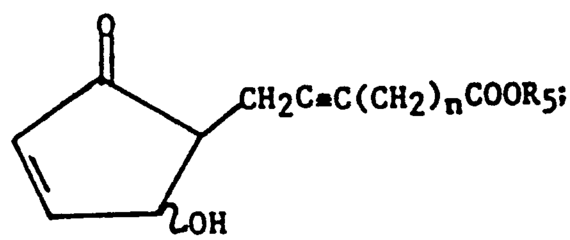

(f) isomerizing the 3-hydroxycyclopentenone produced by step (e) to produce the 4-hydroxycyclopentenone carboxylic acid of the formula

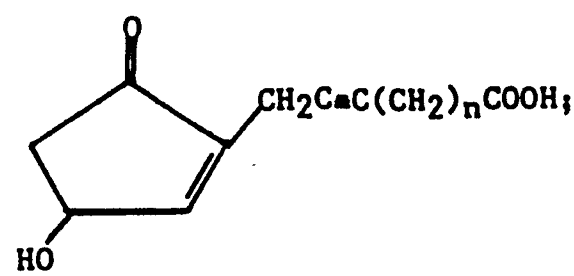

(g) esterifying the carboxylic acid produced in step (f) to produce a compound of the formula

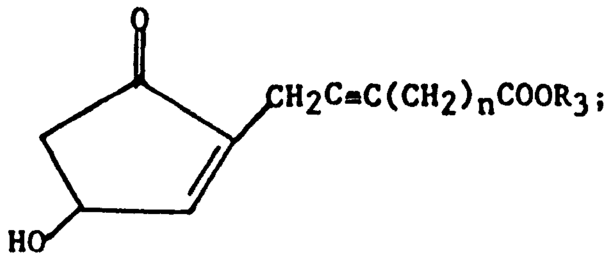

(h) protecting the hydroxy group of the compound produced by step (g) with a blocking group $P_2$ selected from the group consisting of tetrahydropyranyl, trialkylsilyl and alphaalkoxyethyl, giving a compound of the formula

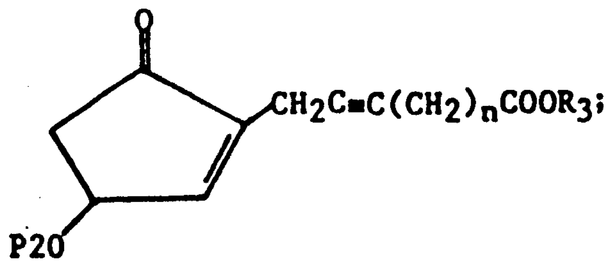

and

(i) subjecting the protected compound produced in step (h) to partial catalytic hydrogenation giving a compound of the formula

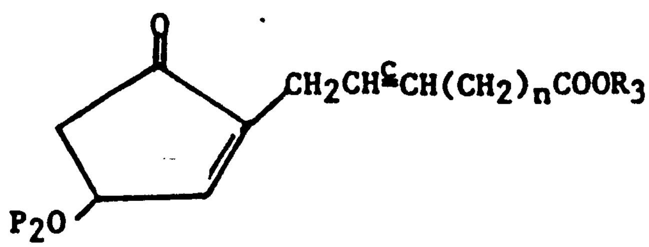

and

(j) subjecting the compound produced step (i) to conjugate addition and hydrolysis to produce the desired compounds.

9. A compound that is a methyl-9-oxo-11 , 16-dihydroxy-16-vinyl-5-cis-13-trans-prostadienoate which contains less than about 2% of the 5-trans stereoisomer.

10. The compound according to Claim 9, having the structure:

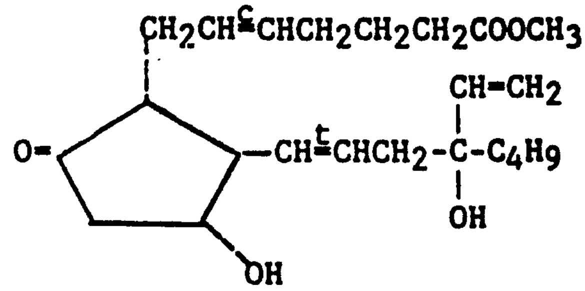

11. A compound made according to the process of Claim 4 or Claim 8 which contains less than about 2% of the 5-trans stereoisomer.

12. A compound according to Claim 11 which is